# FASCICULE DE BREVET EUROPEEN

(11) **EP 1 380 286 B1**
(45) Date de publication et mention de la délivrance du brevet: **01.08.2007**
(21) Numéro de dépôt: 03291451.7
(22) Date de dépôt: 16.06.2003
(51) Int. Cl.: A61Q 5/06, A61K 8/49

(54) **Utilisation pour la teinture des fibres kératiniques humaines d'un chromoionophore et/ou un fluoroionophore, compositions et procédés de teinture**
Verwendung eines Chromoionophoren und/oder eines Fluoroionophoren zum Färben von keratinischen menschlichen Fasern; Zusammensetzungen und Verfahren zum Färben
Use of a chromoionophore and/or a fluoroionophore for dyeing human keratinic fibers, compositions and processes thereof

(30) Priorité: 28.06.2002 FR 0208145
(43) Date de publication de la demande: 14.01.2004
(73) Titulaire: L'ORÉAL, 75008 Paris (FR)
(72) Inventeur: Guerin, Frédéric, 75009 Paris (FR)
(74) Mandataire: Casalonga, Axel

(56) Documents cités:
- EP-A- 0 594 047
- EP-A- 0 826 677
- WO-A-94/04539
- US-A- 4 367 072
- BUSCHMANN H.J., SCHOLLMEYER E.: "Ionenselektive Farbstoffe zum Färben von Polyamid" CHEMIEFASERN/TEXTILINDUSTRIE, vol. 40, no. 5, pages 449-452, XP009006903

## Description

La présente invention concerne le domaine de la teinture des fibres kératiniques humaines telles que les cheveux. Elle concerne plus particulièrement l'utilisation en tant que colorant direct pour la teinture des fibres kératiniques humaines d'un composé choisi parmi les chromoionophores et les fluoroionophores, une composition tinctoriale pour la teinture des fibres kératiniques humaines comprenant, dans un milieu approprié, au moins un tel composé et au moins un adjuvant cosmétique, ainsi que des procédés de teinture desdites fibres et un dispositif à plusieurs compartiments pour la teinture desdites fibres.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres. Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement.

Ces produits de coloration capillaire permettent de changer la couleur des cheveux pendant quelques semaines ou quelques mois. Cependant il peut aussi être intéressant de changer la couleur des cheveux pendant quelques jours voire quelques heures et retrouver la couleur initiale sans altérer l'intégrité de la fibre.

Le problème posé à l'origine de la présente demande est de proposer de nouvelles compositions tinctoriales pour la teinture des fibres kératiniques humaines qui permettent d'obtenir des colorations chromatiques avec des reflets homogènes, tenaces et brillants, et dont la couleur peut ensuite être modifiée simplement, sans ajout d'un autre colorant.

De manière avantageuse et inattendue, la demanderesse a découvert qu'il était possible d'atteindre ce but en utilisant, dans un milieu approprié pour la teinture des fibres kératiniques humaines, au moins un composé choisi parmi les chromoionophores et les fluoroionophores, à titre de colorant direct.

Les compositions tinctoriales selon l'invention permettent l'obtention d'une couleur qui peut ensuite être modifiée pour quelques heures, quelques jours, quelques semaines ou quelques mois. L'utilisateur peut donc choisir une couleur, la modifier en une deuxième couleur puis choisir de revenir ensuite à sa couleur initiale ou conserver la deuxième couleur sans que ces changements de couleur n'entraînent une altération conséquente des cheveux.

Il est ainsi possible selon l'invention de moduler la couleur des chromoionophores/fluoroionophores avant ou après application sur les fibres kératiniques.

Ces compositions selon l'invention permettent l'obtention de couleurs particulièrement chromatiques. Elles peuvent être utilisées pour teindre seulement quelques mèches ou une chevelure complète.

Les teintures obtenues en mettant en oeuvre les compositions tinctoriales selon l'invention sont résistantes aux intempéries, aux lavages et à la transpiration. En outre, les chromoionophores et fluoroionophores sont suffisamment stables en présence d'agents oxydants et réducteurs pour permettre également un éclaircissement de la fibre soit par utilisation de compositions directes éclaircissantes les contenant, soit par l'utilisation de compositions de coloration d'oxydation les contenant. Ces chromoionophores et fluoroionophores permettent de teindre les fibres kératiniques selon une gamme très large de couleurs, en particulier très chromatiques, sans oublier les nuances dites «fondamentales» comme les noirs et les marrons.

Les chromoionophores et fluoroionophores sont des composés constitués d'une ou plusieurs molécules ionophores liées par covalence à au moins une molécule choisie parmi les chromophores et les fluorophores, ladite molécule choisie parmi les chromophores et les fluorophores étant de préférence un colorant direct.

Un premier objet de la présente demande consiste en l'utilisation à titre de colorant direct, dans des, ou pour la fabrication de compositions pour la teinture des fibres kératiniques humaines, et en particulier des cheveux, d'au moins un composé choisi parmi les chromoionophores et les fluoroionophores, ce composé étant constitué d'au moins une molécule ionophore liée par covalence au moins une molécule choisie parmi les chromophores et les fluorophores.

Un deuxième objet de la présente invention concerne une composition tinctoriale pour la teinture des fibres kératiniques humaines, et en particulier des cheveux, comprenant, dans un milieu approprié pour la teinture des fibres kératiniques, au moins un composé choisi parmi les chromoionophores et les fluoroionophores, et au moins un adjuvant cosmétique différent de l'eau, choisi parmi les agents tensio-actifs anioniques, cationiques, non-ioniques, amphotères ou zwittérioniques, ou leurs mélanges, les polymères anioniques, cationiques, non ioniques, amphotères ou zwittérioniques, ou leurs mélanges, les agents épaississants minéraux ou organiques, et en particulier les polymères associatifs anioniques, cationiques, non ioniques et amphotères, les agents de pénétration, les agents séquestrants, les parfums, les agents dispersants, les agents de conditionnement tels que par exemple les silicones volatiles ou non volatiles, modifiées ou non modifiées, les corps gras dont les céramides et les alcools gras, les agents opacifiants.

Par "agent de conditionnement" au sens de la présente demande, on entend un composé susceptible d'améliorer les propriétés cosmétiques des fibres kératiniques telles que le démêlage, le toucher, le lissage, la brillance et le volume.

Un chromophore est un composé qui absorbe dans le visible, ce composé est donc coloré. Parmi les chromophores classiques, ce sont donc ceux qui présentent la propriété d'être des colorants susceptibles de teindre les fibres kératiniques humaines qui sont de préférence utilisés dans le cadre de la présente demande.

Au sens de la présente demande, un fluorophore est un composé qui absorbe dans le domaine des ultra-violets et dans le visible, ce composé est donc coloré. Parmi les fluorophores classiques, ce sont donc ceux qui présentent la propriété d'être des colorants susceptibles de teindre les fibres kératiniques humaines qui sont de préférence utilisés dans le cadre de la présente demande.

De manière préférée, la molécule choisie parmi les chromophores et les fluorophores est un colorant direct comprenant ou non au moins un cycle cycloaliphatique, aromatique ou hétérocyclique. En outre, le colorant direct ne contient pas plus de quatre cycles condensés.

Ces colorants sont notamment choisis parmi les colorants directs classiques. Ce sont des molécules aromatiques ou non aromatiques choisies dans le groupe formé par les colorants directs nitrés benzéniques neutres, acides ou basiques, les colorants directs azoïques neutres, acides ou basiques, les colorants directs quinoniques et en particulier anthraquinoniques neutres, acides ou basiques, les colorants directs aziniques, les colorants directs méthiniques tels que les méthines et les azométhines neutres, acides ou basiques, les colorants directs triarylméthaniques, les colorants directs indoaminiques, les colorants directs naturels tels que les caroténoïdes, les terpénoïdes, les flavonoïdes, les porphyrines, la fluoresceine, la rhodamine, les coumarines.

Les ionophores sont des molécules capables de complexer des ions.

Dans le cadre de la présente demande, tous les ionophores peuvent être utilisés pour former le composé choisi parmi les chromoionophores et les fluoroionophores. La ou les molécules ionophores sont classiquement choisies parmi les molécules à structure ouverte tels que les chélatants ou les podands, les macrocycles appelés éther-couronnes ou coronands, et les macrobicycles appelés cryptands.

Au sens de la présente demande, on entend par " podands ", des molécules à structure ouverte capables de complexer des ions dans lesquels la partie complexante est une chaîne comprenant des hétéroatomes. Ces molécules sont par exemple des oligoéthers. L'article "Chromo- and Fluoroionophores - A new class of dye reagents"- Acc. Chem. Res. 1985, 18, 65-72, à la page 69 et suivantes présente des composés podands qui peuvent être utilisés dans le cadre de la présente demande.

Au sens de la présente demande, on entend par "coronand ", des molécules à structure fermée bidimensionnelles capables de complexer des ions. Généralement ce sont des molécules monocycliques hydrocarbonées contenant des hétéroatomes. Ces molécules sont par exemple des polyéthers macrocycliques comprenant le motif :

- (CH₂-CH₂-Y)ₙ-

dans lequel Y est un hétéroatome choisi parmi O, S, N et P et n est un entier supérieur à 2 et de préférence compris entre 4 et 10.

Ces molécules sont par exemple des éthers couronnes tels que les 15-crown-5, 18-crown-6 ; des benzo-éthers couronnes tels que les benzo-15-crown-5, benzo-18-crown-6, dibenzo-15-crown-5, dibenzo-18-crown-6 ; des monoaza-éthers couronnes ou des diaza-éthers couronnes tels que les aza-15-crown-5, aza-18-crown-6, diaza-15-crown-5, diaza-18-crown-6.

Les éthers couronnes comprennent le motif suivant: 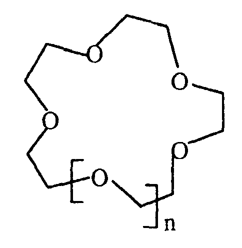 dans lequel n est un nombre entier allant de zéro à 10.

Les monoaza- et diaza-éthers couronnes comprennent les motifs suivants : 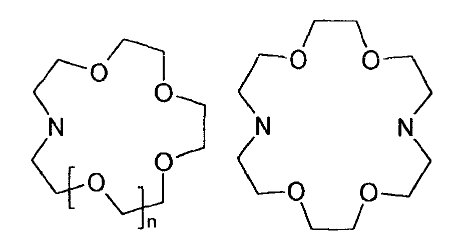 dans lesquels n est un nombre entier allant de zéro à 10.

Au sens de la présente demande, on entend par " cryptands ", des molécules à structure fermée tridimensionnelle (cages) capables de complexer des ions. Généralement ce sont des molécules bicycliques hydrocarbonées contenant des hétéroatomes. Ces molécules sont par exemple des polyéthers macrobicycliques comprenant le motif :

-(CH₂-CH₂-Y)ₙ-

dans lequel Y est un hétéroatome choisi parmi O, S, N et P et n est un entier supérieur à 2 et de préférence compris entre 2 et 10.

Les polyéthers macrobicycliques présentent la structure générale suivante :

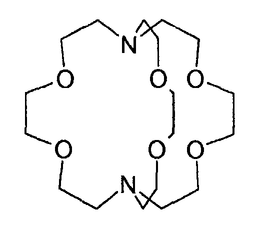

La fixation de la ou des molécules ionophores sur la ou les molécules choisies parmi les chromophores et les fluorophores se fait de préférence par une ou plusieurs liaisons covalentes simples. Cependant, cette fixation peut aussi se faire par un ou plusieurs radicaux divalents tels que des radicaux alkylène (par exemple méthylène, éthylène, propylène, butylène) ou des radicaux divalents aromatiques ou hétérocycliques.

La fixation peut se faire à partir d'un ou plusieurs atomes de la molécule ionophore (atomes de carbone ou hétéroatomes).

Toutes les réactions chimiques classiques peuvent être mises en oeuvre pour préparer les composés choisis parmi les chromoionophores et les fluoroionophores à partir d'une molécule ionophore et d'une ou plusieurs molécules choisies parmi les chromophores et les fluorophores.

Certains des chromoionophores ou des fluoroionophores sont des produits connus en eux-mêmes comme ceux par exemple listés dans le tableau suivant :

| Structure | CAS |
|---|---|
| 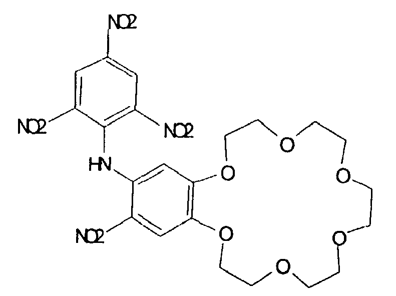 | 74305-50-3 |
| 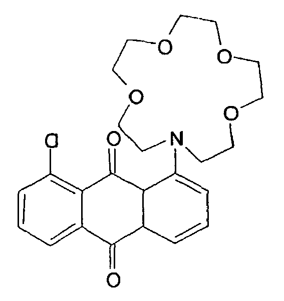 | 77101-97-4 |
| 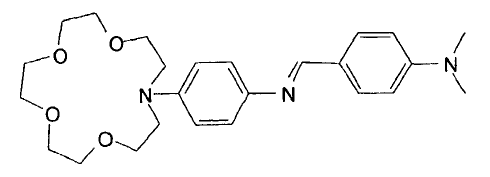 | 361454-18-4 |
| 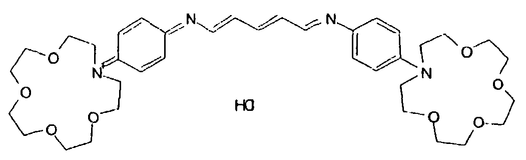 | 73171-31-0 |
| 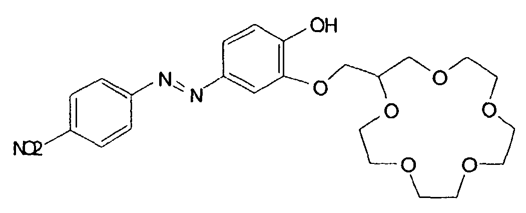 | 81760-15-8 |
| 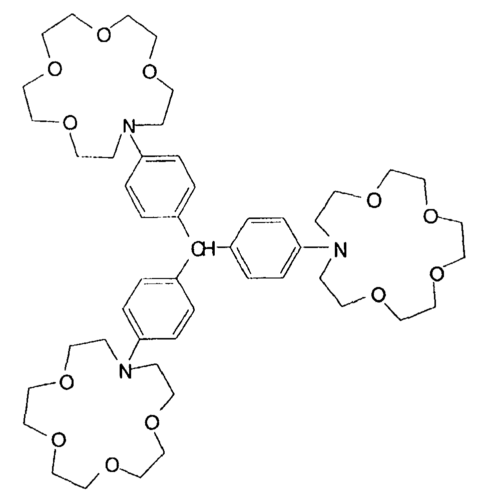 | 66749-97-1 |
| 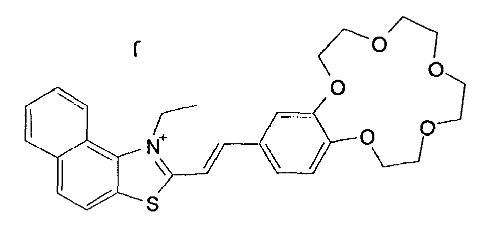 | 174542-39-3 |

Généralement, le ou les composés choisis parmi les chromoionophores et les fluoroionophores sont utilisés en une concentration allant d'environ 0,0001 à 20% en poids par rapport au poids total de la composition, de préférence de 0,0001 à 10% et plus particulièrement de 0,0001 à 5%.

La composition tinctoriale selon la présente invention peut être utilisée pour teindre les fibres kératiniques humaines, et en particulier les cheveux, en une couleur au choix de l'utilisateur qui peut ensuite être modifiée par simple ajout d'un composé ionique pouvant être en outre un adjuvant cosmétique. Ces composés ioniques peuvent être présents dans la composition selon l'invention ou dans une autre composition qu'on applique directement sur les fibres kératiniques humaines.

Sans vouloir être lié à aucune théorie, il apparaît que l'ajout d'ions dans l'environnement des molécules de chromoionophores et/ou fluoroionophores permet de modifier le spectre d'absorption de ces molécules par délocalisation des électrons, ce qui a pour conséquence une modification de la couleur des molécules de chromoionophores et/ou fluoroionophores.

Les ions utilisables selon la présente invention peuvent être des cations ou des anions. De préférence on utilise des cations sous forme de sels.

Les sels utilisés sont des sels physiologiquement acceptables de nature inorganique ou organique.

Parmi les sels inorganiques, les sels métalliques sont préférés, de manière encore plus préférée, ce sont les sels inorganiques de métaux alcalins, de métaux alcalinoterreux, de métaux de transitions, de métaux de terres rares et leurs alliages.

Les métaux alcalins peuvent être choisis parmi le lithium (Li), le sodium (Na), le potassium (K) et le césium (Cs).

Les métaux alcalinoterreux peuvent être choisis parmi le béryllium (Be), le magnésium (Mg), le calcium (Ca), le strontium (Sr) et le baryum (Ba).

Les métaux de transitions peuvent être choisis dans la série des éléments allant du scandium (Sc) au zinc (Zn) tels qu'ils sont présentés dans le tableau périodique des éléments c'est-à-dire parmi les éléments suivants : Sc, Ti, V, Cr, Mn, Fe, Co, Ni, Cu, Zn ; dans la série allant de l'Yttrium (Y) au cadmium (Cd) c'est-à-dire parmi les éléments suivants : Y, Zr, Nb, Mo, Tc, Ru, Rh, Pd, Ag, Cd ; ou de la série du lanthane (La), c'est-à-dire parmi les éléments suivants : La, Hf, Ta, W, Re, Os, Ir, Pt, Au.

Les sels de métaux lourds préférés sont les sels physiologiquement acceptables de zinc, de plomb, de calcium, de cuivre, de manganèse, de fer, de cobalt, de nickel, d'étain, d'argent ou de magnésium, sous formes de sels de chlorures, sulfates, nitrates ou acétates.

Les métaux de terres rares peuvent être choisis dans la série des éléments de type lanthanides, du cérium (Ce) au lutétium (Lu) c'est-à-dire parmi les éléments suivants : Ce, Pr, Nd, Pm, Sm, Eu, Gd, Tb, Dy, Ho, Er, Tm, Yb, Lu.

On utilisera donc dans les compositions de l'invention, les sels correspondants de type nitrate, sulfate, carbonate, halogénure, bromate, phosphate, sulfonate, acétate, chlorure.

Parmi les sels organiques, on préfère les sels d'ammonium tels que le benzyltriméthylammonium, l'isobutylammonium, le butyltriméthylammonium, le décyltriméthylammonium, le diéthylammonium, le diméthylammonium, le dipropylammonium, le dodécylammonium, le dodécyltriméthylammonium, l'éthanolammonium, l'éthylammonium, l'éthyltriméthylammonium, les sels de diammonium.

Le ou les composés ioniques sont présents de préférence à une concentration en poids allant de 0,001 à 25%, de préférence de 0,01 à 10% et plus particulièrement de 0,1 à 5% par rapport au poids total de la composition.

La composition selon la présente invention peut encore comprendre au moins un colorant direct additionnel.

Ce colorant direct additionnel peut être choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

Le ou les colorants directs additionnels représentent de préférence de 0,001 à 20% en poids environ du poids total de la composition et encore plus préférentiellement de 0,005 à 10% en poids environ.

La composition de l'invention peut en outre comprendre un agent oxydant. Cet agent oxydant peut être n'importe quel agent oxydant utilisé de façon classique pour la décoloration des fibres kératiniques. L'agent oxydant est choisi de préférence parmi le peroxyde d'hydrogène, le peroxyde d'urée, les bromates de métaux alcalins, les persels tels que les perborates et les persulfates, les peracides et les enzymes parmi lesquelles on peut citer les peroxydases, les oxy-réductases à 2 électrons telles que les uricases et les oxygénases à 4 électrons comme les laccases. L'utilisation du peroxyde d'hydrogène est particulièrement préférée.

La composition selon l'invention peut en outre comprendre au moins une base d'oxydation. Cette base d'oxydation peut être choisie parmi les bases d'oxydation classiquement utilisées en teinture d'oxydation, par exemple, les paraphénylènediamines, les bis-phénylalkylènediamines, les para-aminophénols, les ortho-aminophénols et les bases hétérocycliques.

La ou les bases d'oxydation sont présentes en une quantité allant de préférence de 0,001 à 10% en poids environ du poids total de la composition tinctoriale, et plus préférentiellement de 0,005 à 6%.

La composition selon l'invention peut contenir en outre un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

Dans la composition de la présente invention, le ou les coupleurs sont en général présents en une quantité allant de 0,001 à 10% en poids environ du poids total de la composition tinctoriale et plus préférentiellement de 0,005 à 6%.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzène-sulfonates, les phosphates et les acétates.

Le milieu approprié pour la teinture appelé aussi support de teinture est généralement constitué par de l'eau ou par un mélange d'eau et d'au moins un solvant organique. A titre de solvant organique, on peut par exemple citer les alcools inférieurs en C₁-C₄, tels que l'éthanol et l'isopropanol ; les polyols et éthers de polyols comme le 2-butoxyéthanol, le propylèneglycol, le monoéthyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants organiques peuvent être présents dans des proportions de préférence comprises entre 1 et 40% en poids environ par rapport au poids total de la composition tinctoriale, et encore plus préférentiellement entre 5 et 30% en poids environ.

Dans la composition tinctoriale conforme à l'invention, les adjuvants cités ci-dessus sont en général présents en une quantité allant pour chacun d'eux de 0,01 à 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 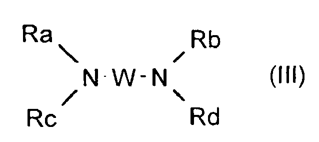 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Un troisième objet de la présente invention consiste en un procédé de teinture des fibres kératiniques humaines, et en particulier des cheveux, qui comprend l'application d'une composition comprenant au moins un composé choisi parmi les chromoionophores et les fluoroionophores, sur les fibres kératiniques humaines pendant une durée suffisante pour développer la coloration désirée. Les fibres sont ensuite rincées puis séchées.

Dans ce procédé, ladite composition peut comprendre en outre un adjuvant cosmétique différent de l'eau qui peut notamment être choisi parmi les agents tensio-actifs anioniques, cationiques, non-ioniques, amphotères, zwittérioniques ou leurs mélanges, les polymères anioniques, cationiques, non ioniques, amphotères ou zwittérioniques ou leurs mélanges, les agents épaississants minéraux ou organiques, et en particulier les polymères associatifs anioniques, cationiques, non ioniques et amphotères, les agents antioxydants, les agents de pénétration, les agents séquestrants, les parfums, les agents dispersants, les agents de conditionnement tels que par exemple les silicones volatiles ou non volatiles, modifiées ou non modifiées, les corps gras dont les céramides et les alcools gras, les agents conservateurs, les agents opacifiants.

Une variante de l'invention consiste à inclure au moins un composé ionique dans la composition comprenant le ou les composés chromoionophores ou fluoroionophores.

Lesdits composés ioniques peuvent alors être présents dans une composition comprenant le ou les chromoionophores ou fluoroionophores, et qui est stockée avant emploi ; ils peuvent aussi être présents dans une seconde composition qu'on ajoute, au moment de l'emploi à celle comprenant les chromoionophores ou fluoroionophores.

Un quatrième objet de la présente invention consiste en un procédé de teinture des fibres kératiniques humaines, en plusieurs étapes, l'une de ces étapes comprenant l'application d'une composition comprenant au moins un composé chromoionophore ou fluoroionophore tel que décrit ci-dessus, ladite étape étant précédée ou suivie par l'application d'au moins une composition comprenant au moins un composé ionique.

L'application des deux compositions peut être séparée par un rinçage intermédiaire.

De préférence, la composition contenant le ou les composés ioniques sera ajoutée sur les fibres kératiniques humaines après l'application du composé choisi parmi les chromoionophores et les fluoroionophores sur ces fibres. De cette façon, l'utilisateur pourra moduler sa couleur initiale de manière plus ou moins importante en ajoutant progressivement des ions.

Chacune des applications dure de 5 minutes à 1 heure, de préférence de 10 à 30 minutes.

Le ou les composés ioniques sont présents dans les compositions de teinture de l'invention en une concentration allant d'environ 0,001 à 25% en poids et de préférence d'environ 0,01 à 10%, et plus préférentiellement encore de 0,1 à 5% par rapport au poids total de la composition.

La composition selon l'invention est généralement appliquée à une température comprise entre la température ambiante et 80°C, de préférence entre 25°C et 55°C.

L'invention a aussi pour objet un procédé de teinture directe éclaircissante qui comprend l'application sur les fibres d'une composition contenant un composé choisi parmi les chromoionophores et les fluoroionophores en présence d'agent oxydant, ce qui provoque la décoloration de la fibre. Cet agent oxydant peut être ajouté à la composition contenant le composé choisi parmi les chromoionophores et les fluoroionophore au moment de l'emploi ou directement sur la fibre kératinique.

L'invention a aussi pour objet un procédé de teinture d'oxydation qui comprend l'application sur les fibres d'une composition tinctoriale qui comprend un composé choisi parmi les chromoionophores et les fluoroionophores, au moins une base d'oxydation et optionnellement au moins un coupleur, en présence d'un agent oxydant. La base d'oxydation, le coupleur et l'agent oxydant sont tels que définis précédemment.

La couleur peut alors être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.

Dans le cas de la teinture directe éclaircissante ou de la teinture d'oxydation, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture d'oxydation des cheveux.

Le pH de la composition oxydante renfermant l'agent oxydant est tel qu'après un mélange avec la composition tinctoriale, le pH de la composition résultante appliquée sur les fibres kératiniques varie de préférence entre 3 et 12 environ, et encore plus préférentiellement entre 5 et 11. Il est peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou toute autre forme appropriée pour réaliser une teinture des fibres kératiniques, et notamment des cheveux humains. Elle peut également être conditionnée sous pression en flacon aérosol en présence d'un agent propulseur et former une mousse.

De la même façon qu'un procédé de teinture directe, dans le cas d'un procédé de teinture directe éclaircissante ou d'un procédé de teinture d'oxydation, l'application de la composition comprenant au moins un composé choisi parmi les chromoionophores et fluoroionophores, peut être précédée ou suivie par l'application d'une composition comprenant au moins un composé ionique.

Un objet supplémentaire de la présente invention est un dispositif à plusieurs compartiments ou "kit" pour la teinture des fibres kératiniques humaines, et plus particulièrement des cheveux. Il comprend au moins deux compartiments, dont l'un d'entre eux renferme une composition comprenant, dans un milieu approprié pour la teinture, au moins un composé choisi parmi les chromoionophores et les fluoroionophores, et un autre une composition comprenant au moins un composé ionique.

L'exemple suivant illustre la présente invention et ne doit être considérés en aucune manière comme limitant l'invention.

La demanderesse a réalisé la composition suivante conforme à l'invention:

| | |
|---|---|
| Colorant ionophore (*) à 10⁻³ mol% | 0,4g |
| Laureth ether sulfate de sodium | 0,1g |
| Eau déminéralisée | Qsp 100g |

Le colorant ionophore (*) a la structure suivante (PM = 414,5) :

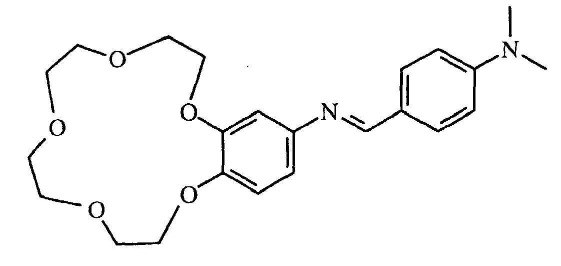

On applique la composition tinctoriale ainsi obtenue sur des mèches de cheveux gris naturels ou permanentés à 90% de blancs et on laisse poser pendant 30 minutes.

Après rinçage à l'eau courante et séchage, les cheveux ont été teints dans une nuance jaune fluorescent.

## Revendications

1. Utilisation à titre de colorant direct, pour la teinture des fibres kératiniques humaines, et en particulier des cheveux, d'au moins un composé choisi parmi les chromoionophores et les fluoroionophores, ce composé étant constitué d'au moins une molécule ionophore liée par covalence à au moins une molécule choisie parmi les chromophores et les fluorophores.

2. Utilisation selon la revendication 1, **caractérisée en ce que** la molécule chromophore ou fluorophore est un colorant direct.

3. Utilisation selon les revendications 1 ou 2, **caractérisée en ce que** la molécule choisie parmi les chromophores et les fluorophores est un colorant direct comprenant ou non au moins un cycle cycloaliphatique, aromatique ou hétérocyclique, et ne comprenant pas plus de quatre cycles condensés.

4. Utilisation selon l'une quelconque des revendications 1 à 3, **caractérisée en ce que** le chromophore ou fluorophore est une molécule choisie dans le groupe formé par les colorants directs nitrés benzéniques neutres, acides ou basiques, les colorants directs azoïques neutres, acides ou basiques, les colorants directs quinoniques et en particulier anthraquinoniques neutres, acides ou basiques, les colorants directs aziniques, les colorants directs méthiniques tels que les méthines et les azométhines neutres, acides ou basiques, les colorants directs triarylméthaniques, les colorants directs indoaminiques, les colorants directs naturels tels que les caroténoïdes, les terpénoïdes, les flavonoïdes, les porphyrines, la fluoresceine, la rhodamine, les coumarines.

5. Utilisation selon l'une quelconque des revendications précédentes, **caractérisée en ce que** la molécule ionophore est choisie parmi les chélatants, les podands, les coronands et les cryptands.

6. Composition tinctoriale pour la teinture des fibres kératiniques humaines, et en particulier des cheveux comprenant, dans un milieu approprié pour la teinture des fibres kératiniques humaines, au moins un adjuvant cosmétique différent de l'eau choisi parmi les agents tensio-actifs anioniques, cationiques, non-ioniques, amphotères ou zwittérioniques, ou leurs mélanges, les polymères anioniques, cationiques, non ioniques, amphotères ou zwittérioniques, ou leurs mélanges, les agents épaississants minéraux ou organiques, et en particulier les polymères associatifs anioniques, cationiques, non ioniques et amphotères, les agents de pénétration, les agents séquestrants, les parfums, les agents dispersants, les agents de conditionnement tels que par exemple les silicones volatiles ou non volatiles, modifiées ou non modifiées, les corps gras dont les céramides et les alcools gras, les agents opacifiants ; et au moins un composé choisi parmi les chromoionophores et les fluoroionophores, ce composé étant constitué d'au moins une molécule ionophore liée par covalence à au moins une molécule choisie parmi les chromophores et les fluorophores.

7. Composition selon l'une quelconque la revendication 6, **caractérisée en ce qu'**elle comprend au moins un composé ionique.

8. Composition selon la revendication 7, **caractérisée en ce que** le composé ionique comprend au moins un cation d'un sel choisi parmi les sels organiques ou inorganiques physiologiquement acceptables.

9. Composition selon la revendication 8, **caractérisée en ce que** le composé ionique est un sel inorganique métallique choisi parmi ceux des métaux alcalins tels que Li, Na, K, Cs, des métaux alcalinoterreux tels que Be, Mg, Ca, Sr, Ba, des métaux de transition tels que Se, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, des métaux de terres rares tels que, Ce, Pr, Nd, Pm, Sm, Eu, Gd, Tb, Dy, Ho, Er, Tm, Yb, Lu, et leurs alliages.

10. Composition selon la revendication 8, **caractérisée en ce que** le composé ionique est un sel organique non-métallique choisi parmi les sels d'ammonium tels que le benzyltriméthylammonium, l'isobutylammonium, le butyltriméthylammonium, le décyltriméthylammonium, le diéthylammonium, le diméthylammonium, le dipropylammonium, le dodécylammonium, le dodécyltriméthylammonium, l'éthanolammonium, l'éthylammonium, l'éthyltriméthylammonium, les sels de diammonium.

11. Composition selon l'une quelconque des revendications 6 à 10, **caractérisée en ce que** la molécule chromophore ou fluorophore est un colorant direct.

12. Composition selon l'une quelconque des revendications 6 à 11, **caractérisée en ce que** le chromophore ou fluorophore est une molécule choisie dans le groupe formé par les colorants directs nitrés benzéniques neutres, acides ou basiques, les colorants directs azoïques neutres, acides ou basiques, les colorants directs quinoniques et en particulier anthraquinoniques neutres, acides ou basiques, les colorants directs aziniques, les colorants directs méthiniques tels que les méthines et les azométhines neutres, acides ou basiques, les colorants directs triarylméthaniques, les colorants directs indoaminiques, les colorants directs naturels tels que les caroténoïdes, les terpénoïdes, les flavonoïdes, les porphyrines, la fluoresceine, la rhodamine, les coumarines.

13. Composition selon l'une quelconque des revendications 6 à 12, **caractérisée en ce que** la molécule ionophore est choisie parmi les chélatants, les podands, les coronands et les cryptands.

14. Composition selon l'une quelconque des revendications 6 à 13, **caractérisée en ce que** le composé choisi parmi les chromoionophores et les fluoroionophores est présent en une concentration allant de 0,0001 à 20%, de préférence de 0,001 à 10% et plus particulièrement de 0,001 à 5% en poids par rapport au poids total de la composition.

15. Composition selon l'une quelconque des revendications 6 à 14, **caractérisée en ce que** le milieu approprié pour la teinture est constitué par de l'eau ou par un mélange d'eau et d'au moins un solvant organique.

16. Composition selon la revendication 15, **caractérisée en ce que** le solvant organique est choisi parmi les alcools inférieurs en C₁-C₄, les polyols et éthers de polyols, et les alcools aromatiques, et leurs mélanges.

17. Composition selon l'une quelconque des revendications 7 à 16, **caractérisée en ce que** le composé ionique est présent à une concentration en poids allant de 0,001 à 25%, de préférence de 0,01 à 10% et plus particulièrement de 0,1 à 5% par rapport au poids total de la composition.

18. Composition selon l'une quelconque des revendications 6 à 17, **caractérisée en ce qu'**elle comprend au moins un colorant direct choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

19. Composition selon la revendication 18, **caractérisée en ce que** le ou les colorants directs sont présents dans une quantité allant de 0,001 % à 10% en poids par rapport au poids total de la composition.

20. Composition selon l'une quelconque des revendications 6 à 19, **caractérisée en ce qu'**elle comprend au moins une base d'oxydation choisie dans le groupe formé par les para-phénylènediamines, les bis-phénylalkylènediamines, les paraaminophénols, les orthoaminophénols et les bases hétérocycliques.

21. Composition selon la revendication 20, **caractérisée en ce que** la ou les bases d'oxydation sont présentes en une quantité allant de 0,001 à 10% en poids par rapport au poids total de la composition.

22. Composition selon la revendication 20 ou 21, **caractérisée en ce qu'**elle comprend au moins un coupleur choisi dans le groupe formé par les métaphénylènediamines, les méta-aminophénols, les méta-diphénols, les coupleurs naphtaléniques et les coupleurs hétérocycliques.

23. Composition selon la revendication 22, **caractérisée en ce que** le ou les coupleurs sont présents en une quantité allant de 0,001 à 10% en poids par rapport au poids total de la composition.

24. Composition selon l'une des revendications 6 à 23, **caractérisée en ce qu'**elle comprend au moins un agent oxydant, de préférence choisi parmi le peroxyde d'hydrogène, le peroxyde d'urée, les bromates de métaux alcalins, les persels tels que les perborates et les persulfates, les peracides et les enzymes telles que les peroxydases, les oxydo-réductases à 2 électrons et les oxygénases à 4 électrons.

25. Procédé de teinture des fibres kératiniques humaines et en particulier des cheveux, **caractérisé en ce que** l'on applique sur lesdites fibres kératiniques humaines une composition comprenant au moins un composé choisi parmi les chromoionophores et les fluoroionophores, pendant une durée suffisante pour développer la coloration désirée, ce composé étant constitué d'au moins une molécule ionophore liée par covalence à au moins une molécule choisie parmi les chromophores et les fluorophores.

26. Procédé de teinture des fibres kératiniques humaines, et en particulier des cheveux, selon la revendication 25, **caractérisé en ce que** ladite composition comprend en outre un composé ionique.

27. Procédé de teinture des fibres kératiniques humaines, et en particulier des cheveux, selon la revendication 25, **caractérisé en ce que** l'on applique ensuite sur lesdites fibres kératiniques humaines une composition comprenant au moins un composé ionique.

28. Procédé de teinture des fibres kératiniques humaines, et en particulier des cheveux selon les revendications 25, **caractérisé en ce que** l'on applique au préalable sur lesdites fibres kératiniques humaines, une composition comprenant au moins un composé ionique.

29. Procédé de teinture des fibres kératiniques humaines, et en particulier des cheveux, selon les revendications 26 à 28, **caractérisé en ce que** les composés ioniques sont choisis parmi les sels inorganiques métalliques de métaux alcalins tels que Li, Na, K, Cs, de métaux alcalinoterreux tels que Be, Mg, Ca, Sr, Ba, de métaux de transitions tels que Sc, Ti, V, Cr, Mn, Fe, Co, Ni, Cu, Zn, Y, Zr, Nb, Mo, Tc, Ru, Rh, Pd, Ag, La, Cd, Hf, Ta, W, Re, Os, Ir, Pt, Au, de métaux de terres rares tels que Ce, Pr, Nd, Pm, Sm, Eu, Gd, Tb, Dy, Ho, Er, Tm, Yb, Lu et leurs alliages, et les sels organiques non métalliques choisis parmi les sels d'ammonium tels que le benzyltriméthylammonium, l'isobutylammonium, le butyltriméthylammonium, le décyltriméthylammonium, le diéthylammonium, le diméthylammonium, le dipropylammonium, le dodécylammonium, le dodécyltriméthylammonium, l'éthanolammonium, l'éthylammonium, l'éthyltriméthylammonium, les sels de diammonium.

30. Dispositif à plusieurs compartiments ou "kit" pour la teinture des fibres kératiniques humaines, et plus particulièrement des cheveux, **caractérisé en ce qu'**il comprend au moins deux compartiments, dont l'un d'entre eux renferme une composition comprenant, dans un milieu approprié pour la teinture, au moins un composé choisi parmi les chromoionophores et les fluoroionophores, et un autre une composition comprenant au moins un composé ionique.

## Claims

1. Use as direct dye, for dyeing human keratin fibres, hair in particular, of at least one compound chosen from among the chromoionophores and fluoroionophores, this compound being made up of at least one ionophore molecule covalently bound to at least one molecule chosen from among the chromophores and the fluorophores.

2. Use according to claim 1, **characterized in that** the chromophore or fluorophore molecule is a direct dye.

3. Use according to claims 1 or 2, **characterized in that** the molecule chosen from among the chromophores and fluorophores is a direct dye, comprising or not comprising at least one cycloaliphatic, aromatic or heterocyclic cycle, and not comprising more than four condensed cycles.

4. Use according to any of claims 1 to 3, **characterized in that** the chromophore or fluorophore is a molecule chosen from the group consisting of neutral, acid or basic nitrobenzene direct dyes, neutral, acid or basic azoic direct dyes, quinone direct dyes and in particular neutral, acid or basic anthraquinone direct dyes, azine direct dyes, methine direct dyes such as neutral, acid or basic methines and azomethines, triarylmethane direct dyes, indoamine direct dyes, natural direct dyes such as carotenoids, terpenoids, flavonoids, porphyrins, fluorescein, rhodamine, coumarins.

5. Use according to any of the preceding claims, **characterized in that** the ionophore molecule is chosen from among chelatants, podands, coronands and cryptands.

6. Dye composition for dyeing human keratin fibres, hair in particular, comprising - in an appropriate medium for dyeing human keratin fibres - at least one cosmetic additive other than water chosen from among anionic, cationic, nonionic, amphoteric or zwitterionic surfactants or their mixtures, anionic, cationic, nonionic, amphoteric or zwitterionic polymers or their mixtures, mineral or organic thickening agents, in particular anionic, cationic, nonionic and amphoteric associative polymers, penetration agents, sequestering agents, perfumes, dispersing agents, conditioning agents such as for example modified or not modified volatile or non-volatile silicones, fats including ceramides and fatty alcohols, opacifying agents; and at least one compound chosen from among the chromoionophores and the fluoroionophores, this compound being made up of at least one ionophore molecule covalently bound to at least one molecule chosen from among the chromophores and the fluorophores.

7. Composition according to claim 6, **characterized in that** it contains at least one ionic compound.

8. Composition according to claim 7, **characterized in that** the ionic compound contains at least one cation of a salt chosen from physiologically acceptable organic or inorganic salts.

9. Composition according to claim 8, **characterized in that** the ionic compound is an inorganic metal salt chosen from among those of alkaline metals such as Li, Na, K, Cs, of alkaline earth metals such as Be, Mg, Ca, Sr, Ba, of transition metals such as Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, of rare earth metals such as Ce, Pr, Nd, Pm, Sm, Eu, Gd, Tb, Dy, Ho, Er, Tm, Yb, Lu and their alloys.

10. Composition according to claim 8, **characterized in that** the ionic compound is a non-metallic organic salt chosen from among the ammonium salts such as benzyltrimethylammonium, isobutylammonium, butyltrimethylammonium, decyltrimethylammonium, diethylammonium, dimethylammonium, dipropylammonium, dodecylammonium, dodecyltrimethylammonium, ethanol-ammonium, ethylammonium, ethyltrimethylammonium, diammonium salts.

11. Composition according to any of claims 6 to 10, **characterized in that** the chromophore or fluorophore molecule is a direct dye.

12. Composition according to any of claims 6 to 11, **characterized in that** the chromophore or fluorophore is a molecule chosen from the group consisting of neutral, acid or basic nitrobenzene direct dyes, neutral, acid or basic azoic direct dyes, quinone direct dyes and in particular neutral, acid or basic anthraquinone direct dyes, azine direct dyes, methine direct dyes such as neutral, acid or basic methines and azomethines, triarylmethane direct dyes, indoamine direct dyes, natural direct dyes such as carotenoids, terpenoids, flavonoids, porphyrins, fluorescein, rhodamine, coumarins.

13. Composition according to any of claims 6 to 12, **characterized in that** the ionophore molecule is chosen from among chelatants, podands, coronands and cryptands.

14. Composition according to any of claims 6 to 13, **characterized in that** the compound chosen from among the chromoionophores and the fluoroionophores is present at a concentration ranging from 0.0001 to 20 %, preferably from 0.001 to 10 %, and further preferably from 0.001 to 5 % by weight relative to the total weight of the composition.

15. Composition according to any of claims 6 to 14, **characterized in that** the appropriate medium for dyeing is formed of water or a mixture of water and at least one organic solvent.

16. Composition according to claim 15, **characterized in that** the organic solvent is chosen from among lower C₁-C₄ alcohols, polyols and polyol ethers, aromatic alcohols, and mixtures thereof.

17. Composition according to any of claims 7 to 16, **characterized in that** the ionic compound is present in a weight concentration ranging from 0.001 to 25 %, preferably from 0.01 to 10 % and more particularly from 0.1 to 5 % relative to the total weight of the composition.

18. Composition according to any of claims 6 to 17, **characterized in that** it comprises at least one direct dye chosen from among neutral, acid or cationic nitrobenzene direct dyes, neutral, acid or cationic azoic direct dyes, quinone direct dyes and in particular neutral, acid or cationic anthraquinone direct dyes, azine direct dyes, methine direct dyes, triarylmethane direct dyes, indoamine direct dyes and natural direct dyes.

19. Composition according to claim 18, **characterized in that** the direct dye or dyes are present in a quantity ranging from 0.001 % to 10 % by weight relative to the total weight of the composition.

20. Composition according to any of claims 6 to 19, **characterized in that** it comprises at least one oxidation base chosen from the group consisting of para-phenylenediamines, bis-phenylalkylenediamines, paraaminophenols, orthoaminophenols and heterocyclic bases.

21. Composition according to claim 20, **characterized in that** the oxidation base or bases are present in a quantity ranging from 0.001 to 10 % by weight relative to the total weight of the composition.

22. Composition according to claim 20 or 21, **characterized in that** it comprises at least one coupler chosen from the group consisting of metaphenylenediamines, metaaminophenols, metadiphenols, naphthalene couplers and heterocyclic couplers.

23. Composition according to claim 22, **characterized in that** the coupler or couplers are present in a quantity ranging from 0.001 to 10 % by weight relative to the total weight of the composition.

24. Composition according to any of claims 6 to 23, **characterized in that** it comprises at least one oxidizing agent, preferably chosen from among hydrogen peroxide, urea peroxide, alkaline metals bromates, persalts such as perborates and persulphates, peracids, and enzymes such as peroxydases, 2-electron oxido-reductases and 4-electron oxygenases.

25. Method for dyeing human keratin fibres, hair in particular, **characterized in that** a composition is applied to said human keratin fibres, such composition comprising at least one compound chosen from among the chromoionophores and the fluoroionophores for a length of time sufficient to develop the desired colour, this compound being made up of at least one ionophore molecule covalently bound to at least one molecule chosen from among the chromophores and the fluorophores.

26. Method for dyeing human keratin fibres, hair in particular, according to claim 25, **characterized in that** said composition also comprises an ionic compound.

27. Method for dyeing human keratin fibres, hair in particular, according to claim 25, **characterized in that** a composition comprising at least one ionic compound is subsequently applied to said human keratin fibres.

28. Method for dyeing human keratin fibres, hair in particular, according to claim 25 **characterized in that** a composition comprising at least one ionic compound is previously applied to said human keratin fibres.

29. Method for dyeing human keratin fibres, hair in particular, according to claims 26 to 28, **characterized in that** the ionic compounds are chosen from the inorganic metal salts of alkaline metals such as Li, Na, K, Cs, of alkaline earth metals such as Be, Mg, Ca, Sr, Ba, of transition metals such as Sc, Ti, V, Cr, Mn, Fe, Co, Ni, Cu, Zn, Y, Zr, Nb, Mo, Tc, Ru, Rh, Pd, Ag, La, Cd, Hf, Ta, W, Re, Os, Ir, Pt, Au, of rare earth metals such as Ce, Pr, Nd,Pm, Sm, Eu, Gd, Tb, Dy, Ho, Er, Tm, Yb, Lu and their alloys, and the nonmetallic organic salts chosen from among the ammonium salts such as benzyltrimethylammonium, isobutylammonium, butyltrimethylammonium, decyltrimethylammonium, diethylammonium, dimethylammonium, dipropylammonium, dodecylammonium, dodecyltrimethylammonium, ethanol-ammonium, ethylammonium, ethyltrimethylammonium, diammonium salts.

30. Device with several compartments or "kit" for dyeing human keratin fibres, and more especially hair, **characterized in that** it comprises at least two compartments, of which one contains a composition comprising - in an appropriate dyeing medium - at least one compound chosen from among the chromoionophores and the fluoroionophores, and another contains a composition comprising at least one ionic compound.

## Patentansprüche

1. Verwendung mindestens einer Verbindung, die unter den Chromoionophoren und Fluoroionophoren ausgewählt ist, als Direktfarbstoff zum Färben von menschlichen Keratinfasern und insbesondere Haaren, wobei diese Verbindung aus mindestens einem Ionophor besteht, das kovalent an mindestens ein Molekül gebunden ist, das unter den Chromophoren und Fluorophoren ausgewählt ist.

2. Verwendung nach Anspruch 1, **dadurch gekennzeichnet, dass** das chromophore Molekül oder fluorophore Molekül ein Direktfarbstoff ist.

3. Verwendung nach Anspruch 1 oder 2, **dadurch gekennzeichnet, dass** das Molekül, das unter den Chromophoren und Fluorophoren ausgewählt ist, ein Direktfarbstoff ist, der gegebenenfalls mindestens einen cycloaliphatischen, aromatischen oder heterocyclischen Ring aufweist und nicht mehr als vier kondensierte Ringe umfasst.

4. Verwendung nach einem der Ansprüche 1 bis 3, **dadurch gekennzeichnet, dass** das Chromophor oder Fluorophor ein Molekül ist, das unter den neutralen, sauren oder basischen, direktziehenden nitrierten Benzolfarbstoffen, neutralen, sauren oder basischen direktziehenden Azofarbstoffen, direktziehenden Chinon-Farbstoffen und insbesondere neutralen, sauren oder basischen Antrachinon-Farbstoffen, direktziehenden Azin-Farbstoffen, direktziehenden Methin-Farbstoffen, wie neutralen, sauren oder basischen Methinen und Azomethinen, direktziehenden Triarylmethan-Farbstoffen, direktziehenden Indoamin-Farbstoffen, direktziehenden natürlichen Farbstoffen, wie Carotinoiden, Terpenoiden, Flavonoiden, Porphyrinen, Fluorescin, Rhodamin und Cumarinen ausgewählt ist.

5. Verwendung nach einem der vorhergehenden Ansprüche, **dadurch gekennzeichnet, dass** das Ionophor unter den Chelatbildnern, Podanden, Koronanden und Kryptanden ausgewählt ist.

6. Farbmittelzusammensetzung zum Färben von menschlichen Keratinfasern und insbesondere Haaren, die in einem zum Färben von menschlichen Keratinfasern geeigneten Medium mindestens ein von Wasser verschiedenes kosmetisches Adjuvans enthält, das unter den anionischen, kationischen, nichtionischen, amphoteren oder zwitterionischen grenzflächenaktiven Stoffen oder ihren Gemischen, anionischen, kationischen, nichtionischen, amphoteren oder zwitterionischen Polymeren oder deren Gemischen, anorganischen oder organischen Verdickungsmitteln und insbesondere anionischen, kationischen, nichtionischen und amphoteren assoziativen Polymeren, Penetrationsmitteln, Maskierungsmitteln, Parfums, Dispergiermitteln, Konditioniermitteln, wie beispielsweise flüchtigen oder nicht flüchtigen, modifizierten oder nichtmodifizierten Siliconen, Fettstoffen und darunter Ceramiden und Fettalkoholen, Trübungsmitteln ausgewählt ist; und mindestens eine Verbindung enthält, die unter den Chromoionophoren und Fluoroionophoren ausgewählt ist, wobei die Verbindung aus mindestens einem Ionophor besteht, das kovalent an mindestens ein Molekül gebunden ist, das unter den Chromophoren und Fluorophoren ausgewählt ist.

7. Zusammensetzung nach Anspruch 6, **dadurch gekennzeichnet, dass** sie mindestens eine ionische Verbindung enthält.

8. Zusammensetzung nach Anspruch 7, **dadurch gekennzeichnet, dass** die ionische Verbindung mindestens ein Kation eines Salzes umfasst, das unter den physiologisch verträglichen, organischen oder anorganischen Salzen ausgewählt ist.

9. Zusammensetzung nach Anspruch 8, **dadurch gekennzeichnet, dass** die ionische Verbindung ein anorganisches Metallsalz ist, das unter den Salzen von Alkalimetallen, wie Li, Na, K, Cs, von Erdalkalimetallen, wie Be, Mg, Ca, Sr, Ba, von Übergangsmetallen, wie Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, von Seltenerdmetallen, wie Ce, Pr, Nd, Pm, Sm, Eu, Gd, Tb, Dy, Ho, Er, Tm, Yb, Lu, und ihren Legierungen ausgewählt ist.

10. Zusammensetzung nach Anspruch 8, **dadurch gekennzeichnet, dass** die ionische Verbindung ein organisches, nicht metallhaltiges Salz ist, das unter den Salzen von Ammonium, wie Benzyltrimethylammonium, Isobutylammonium, Butyltrimethylammonium, Decyltrimethylammonium, Diethylammonium, Dimethylammonium, Dipropylammonium, Dodecylammonium, Dodecyltrimethylammonium, Ethanolammonium, Ethylammonium, Ethyltrimethylammonium, und Salzen von Diammonium ausgewählt ist.

11. Zusammensetzung nach einem der Ansprüche 6 bis 10, **dadurch gekennzeichnet, dass** das Chromophor oder Fluorophor ein Direktfarbstoff ist.

12. Zusammensetzung nach einem der Ansprüche 6 bis 11, **dadurch gekennzeichnet, dass** das Chromophor oder Fluorophor ein Molekül ist, das unter den neutralen, sauren oder basischen, direktziehenden nitrierten Benzolfarbstoffen, neutralen, sauren oder basischen direktziehenden Azofarbstoffen, direktziehenden Chinon-Farbstoffen und insbesondere neutralen, sauren oder basischen Antrachinon-Farbstoffen, direktziehenden Azin-Farbstoffen, direktziehenden Methin-Farbstoffen, wie neutralen, sauren oder basischen Methinen und Azomethinen, direktziehenden Triarylmethan-Farbstoffen, direktziehenden Indoamin-Farbstoffen, direktziehenden natürlichen Farbstoffen, wie Carotinoiden, Terpenoiden, Flavonoiden, Porphyrinen, Fluorescin, Rhodamin und Cumarinen ausgewählt ist.

13. Zusammensetzung nach einem der Ansprüche 6 bis 12, **dadurch gekennzeichnet, dass** das Ionophor unter den Chelatbildnern, Podanden, Koronanden und Kryptanden ausgewählt ist.

14. Zusammensetzung nach einem der Ansprüche 6 bis 13, **dadurch gekennzeichnet, dass** die Verbindung, die unter den Chromoionophoren und Fluoroionophoren ausgewählt ist, in einer Konzentration von 0,0001 bis 20 Gew.-%, vorzugsweise 0,001 bis 10 Gew.-% und insbesondere 0,001 bis 5 Gew.-%, bezogen auf das Gesamtgewicht der Zusammensetzung, enthalten ist.

15. Zusammensetzung nach einem der Ansprüche 6 bis 14, **dadurch gekennzeichnet, dass** das zum Färben geeignete Medium aus Wasser oder einem Gemisch von Wasser und mindestens einem organischen Lösungsmittel besteht.

16. Zusammensetzung nach Anspruch 15, **dadurch gekennzeichnet, dass** das organische Lösungsmittel unter den niederen C₁-₄-Alkoholen, den Polyolen und Polyolethern und den aromatischen Alkoholen und deren Gemischen ausgewählt ist.

17. Zusammensetzung nach einem der Ansprüche 7 bis 16, **dadurch gekennzeichnet, dass** die ionische Verbindung in einer Konzentration von 0,001 bis 25 Gew.-%, vorzugsweise 0,01 bis 10 Gew.-% und insbesondere 0,1 bis 5 Gew.-%, bezogen auf das Gesamtgewicht der Zusammensetzung, enthalten ist.

18. Zusammensetzung nach einem der Ansprüche 6 bis 17, **dadurch gekennzeichnet, dass** sie mindestens einen Direktfarbstoff enthält, der unter den neutralen, sauren oder kationischen direktziehenden nitrierten Benzolfarbstoffen, neutralen, sauren oder kationischen direktziehenden Azofarbstoffen, direktziehenden Chinon-Farbstoffen und insbesondere neutralen, sauren oder kationischen Anthrachinon-Farbstoffen, direktziehenden Azin-Farbstoffen, direktziehenden Methin-Farbstoffen, direktziehenden Triarylmethan-Farbstoffen, direktziehenden Indoamin-Farbstoffen und natürlichen direktziehenden Farbstoffen ausgewählt ist.

19. Zusammensetzung nach Anspruch 18, **dadurch gekennzeichnet, dass** der oder die Direktfarbstoff(e) in einer Menge von 0,001 bis 10 Gew.-%, bezogen auf das Gesamtgewicht der Zusammensetzung, enthalten sind.

20. Zusammensetzung nach einem der Ansprüche 6 bis 19, **dadurch gekennzeichnet, dass** sie mindestens eine Oxidationsbase enthält, die unter den *p*-Phenylendiaminen, Bis-phenylalkylendiaminen, *p*-Aminophenolen, o-Aminophenolen und heterocyclischen Basen ausgewählt ist.

21. Zusammensetzung nach Anspruch 20, **dadurch gekennzeichnet, dass** die Oxidationsbase(n) in einer Menge von 0,001 bis 10 Gew.-%, bezogen auf das Gesamtgewicht der Zusammensetzung, enthalten sind.

22. Zusammensetzung nach Anspruch 20 oder 21, **dadurch gekennzeichnet, dass** sie mindestens einen Kuppler enthält, der unter den *m*-Phenylendiaminen, *m*-Aminophenolen, *m*-Dihydroxybenzolen, Naphthalinkupplern und heterocyclischen Kupplern ausgewählt ist.

23. Zusammensetzung nach Anspruch 22, **dadurch gekennzeichnet, dass** der oder die Kuppler in Mengenanteilen von 0,001 bis 10 Gew.-%, bezogen auf das Gesamtgewicht der Zusammensetzung, enthalten sind.

24. Zusammensetzung nach einem der Ansprüche 6 bis 23, **dadurch gekennzeichnet, dass** sie mindestens ein Oxidationsmittel enthält, das vorzugsweise unter Wasserstoffperoxid, Harnstoffperoxid, Alkalimetallbromaten, Salzen von Persäuren, wie Perboraten und Persulfaten, Persäuren und Enzymen, wie Peroxydasen, Oxidoreduktasen (2 Elektronen) und Oxygenasen (4 Elektronen), ausgewählt ist.

25. Verfahren zum Färben von menschlichen Keratinfasern und insbesondere Haaren, **dadurch gekennzeichnet, dass** auf die menschlichen Keratinfasern eine Zusammensetzung, die mindestens eine Verbindung enthält, die unter den Chromoionophoren und Fluoroionophoren ausgewählt ist, während einer Zeitspanne aufgebracht wird, die ausreichend ist, um die gewünschte Färbung zu bilden, wobei die Verbindung aus mindestens einem Ionophor besteht, das kovalent an mindestens ein Molekül gebunden ist, das unter den Chromophoren und Fluorophoren ausgewählt ist.

26. Verfahren zum Färben von menschlichen Keratinfasern und insbesondere Haaren nach Anspruch 25, **dadurch gekennzeichnet, dass** die Zusammensetzung ferner eine ionische Verbindung enthält.

27. Verfahren zum Färben von menschlichen Keratinfasern und insbesondere Haaren nach Anspruch 25, **dadurch gekennzeichnet, dass** anschließend auf die menschlichen Keratinfasern eine Zusammensetzung aufgetragen wird, die mindestens eine ionische Verbindung enthält.

28. Verfahren zum Färben von menschlichen Keratinfasern und insbesondere Haaren nach Anspruch 25, **dadurch gekennzeichnet, dass** vorab eine Zusammensetzung, die mindestens eine ionische Verbindung enthält, auf die menschlichen Keratinfasern aufgebracht wird.

29. Verfahren zum Färben von menschlichen Keratinfasern und insbesondere Haaren nach den Ansprüchen 26 bis 28, **dadurch gekennzeichnet, dass** die ionischen Verbindungen unter den anorganischen Metallsalzen von Alkalimetallen, wie Li, Na, K, Cs, von Erdalkalimetallen, wie Be, Mg, Ca, Sr, Ba, von Übergangsmetallen, wie Sc, Ti, V, Cr, Mn, Fe, Co, Ni, Cu, Zn, Y, Zr, Nb, Mo, Tc, Ru, Rh, Pd, Ag, La, Cd, Hf, Ta, W, Re, Os, Ir, Pt, Au, von Seltenerdmetallen, wie Ce, Pr, Nd, Pm, Sm, Eu, Gd, Tb, Dy, Ho, Er, Tm, Yb, Lu, und deren Legierungen und organischen, nicht metallhaltigen Salzen ausgewählt sind, die unter den Ammoniumsalzen, wie Benzyltrimethylammonium, Isobutylammonium, Butyltrimethylammonium, Decyltrimethylammonium, Diethylammonium, Dimethylammonium, Dipropylammonium, Dodecylammonium, Dodecyltrimethylammonium, Ethanolammonium, Ethylammonium, Ethyltrimethylammonium, und Diammoniumsalzen ausgewählt sind.

30. Vorrichtung mit mehreren Abteilungen oder "Kit" zum Färben von menschlichen Keratinfasern und insbesondere Haaren, **dadurch gekennzeichnet, dass** sie mindestens zwei Abteilungen aufweist, wobei eine Abteilung eine Zusammensetzung enthält, die in einem zum Färben geeigneten Medium mindestens eine Verbindung enthält, die unter den Chromoionophoren und Fluoroionophoren ausgewählt ist, und eine andere Abteilung eine Zusammensetzung mit mindestens einer ionischen Verbindung enthält.
